(19) Europäisches Patentamt
European Patent Office
Office européen des brevets

(11) **EP 2 159 278 A1**

(12) **EUROPEAN PATENT APPLICATION**

| | |
|---|---|
| (43) Date of publication:<br>**03.03.2010 Bulletin 2010/09** | (51) Int Cl.:<br>***C12N 9/04*** *(2006.01)*     ***C11D 3/386*** *(2006.01)* |

(21) Application number: **09155707.4**

(22) Date of filing: **20.03.2009**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK TR**<br>Designated Extension States:<br>**AL BA RS**<br><br>(30) Priority: **27.08.2008 US 190305**<br><br>(71) Applicant: **THE PROCTER & GAMBLE COMPANY**<br>**Cincinnati, Ohio 45202 (US)** | (72) Inventor: **Lant, Neil Joseph**<br>**Newcastle upon Tyne, Tyne and Wear NE3 5 (GB)**<br><br>(74) Representative: **Howard, Phillip Jan**<br>**Procter & Gamble**<br>**Technical Centres Limited**<br>**Whitley Road**<br>**Longbenton**<br>**Newcastle upon Tyne NE12 9TS (GB)** |

(54) **A detergent composition comprising carbohydrate: acceptor oxidoreductase**

(57) The present invention relates to a detergent composition comprising carbohydrate:acceptor oxidoreductase.

**Description**

FIELD OF THE INVENTION

The present invention relates to detergent compositions comprising carbohydrate:acceptor oxidoreductase. The compositions of the present invention exhibit good bleaching performance. Preferably, the compositions are laundry detergent compositions, most preferably liquid laundry detergent compositions.

BACKGROUND OF THE INVENTION

The incorporation of bleach into a detergent composition requires a carefully balance of having good bleaching performance and good bleach stability, especially good product storage stability. Whilst many different types of bleach have been considered for incorporation into detergent compositions, such as pre-formed peracids, transition metal bleach catalysts, nitrile quaternary amine bleach activators, cationic imine bleach boosting compounds and the like, there remains very few bleach technologies that exhibit sufficient bleach stability, especially good product storage stability, whilst at the same time providing good bleaching performance and acceptable fabric integrity profile. This is especially true for liquid laundry detergent compositions. Even after decades of development, the commercial yardstick for bleach profile in a solid laundry detergent composition remains, with very few exceptions, percarbonate and/or perborate sources of hydrogen peroxide in combination with bleach activators such as tetraacetylethylenediamine (TAED) and/or alkyloxybenezenesulphonate (AOBS).

With the recent trend in detergent formulation towards increased reliance on improved weight-efficient and cost-effective technologies, developments of bleach catalysis has resurfaced and bleach catalyst technologies such as transition metal catalysts and cationic imine bleach boosting compounds have rekindled. However, these bleach catalyst technologies are used in relatively very small amounts, due to their high weight efficiency.

Using bleach catalysts at these very low levels means that their activity is easily depleted by other components of the detergent composition, such as polyamines. Further instability can arise if the catalysts prematurely activate during storage. This is especially true when the detergent composition is a laundry detergent composition due to the complexity and numerous other chemical components present therein. Also, the bleach stability of the bleach catalyst, and the need to avoid any bleach depletion, is even more critical when the detergent composition is in liquid form, such as a liquid laundry detergent composition.

In addition, using bleach catalysts at very low levels means that it is important that the catalysts are active in the wash when and where bleaching performance is needed. This typically means at the surface to be cleaned. If the bleach catalyst does not reach the surface to be cleaned in any appreciable amount but instead remains in the wash liquor and catalyses bleaching performance in the wash solution, this solution bleaching performance is usually not very efficient as the majority of the available oxygen will be used to bleach soils that are already suspended in the wash liquor and only a minority of the available oxygen will be used to bleach soils that are present at the surface and contribute to the cleaning performance of the detergent composition. This is especially true when the composition is a laundry detergent composition and the surface to be cleaned is a fabric surface.

The Inventor has overcome the above problems and has found a bleach catalyst enzyme that has good stability profile, provides excellent bleaching performance, and exhibits very good bleach efficiency. The inventor has found that a specific carbohydrate:acceptor oxidoreductase generates available oxygen at the fabric surface during a laundering process: the substrate for this specific bleaching enzyme are oligosaccharides arising from cotton as well as other reducing sugars, such as glucose and lactose, present in the fabric stains.

The Inventor has also found that this enzyme provides excellent whiteness benefits due to its effect on the cotton fabric surface which makes the cotton more resistant to soil re-deposition. Without wishing to be bound by theory, the Inventor believes the enzymes acts so as to make the cotton surface more negatively charged (e.g. the enzyme reacts with the terminal glucose moieties of amorphous cellulose and other oligosaccharides of cotton) by oxidizing the terminal reducing ends to a gluconic acid form. This increases the negative charge of the cotton, leading to improved soil repulsion and improved soil anti-redeposition properties of the modified cotton.

SUMMARY OF THE INVENTION

The present invention provides a composition according to the claims.

DETAILED DESCRIPTION OF THE INVENTION

Detergent composition

The detergent composition can be any detergent composition, such as a laundry detergent composition, dishwashing detergent composition, hard-surface cleaning detergent composition, and the like. Preferably, the composition is a laundry detergent composition.

The composition can be in any form, such as a solid or a liquid. Solid forms typically include granular, flake, noodle, needle and the like. Alternatively the composition can in be the form of a gel, paste, suspension or the like. Preferably, the composition is in the form of a liquid. Most preferably, the composition is a liquid laundry detergent composition.

The composition typically comprises detergent adjunct ingredients.

Carbohydrate: acceptor oxidoreductase

The composition comprises carbohydrate:acceptor oxidoreductase. Preferably, the composition comprises from 0.00015wt% to 0.50wt% carbohydrate:acceptor oxidoreductase.

Preferably, the carbohydrate:acceptor oxidoreductase is a parent or variant of the carbohydrate:acceptor oxidoreductase derived from the organism *Paraconiothyrium* sp.. A suitable carbohydrate:acceptor oxidoreductase and its purification method is disclosed in T.Kiryu et al, Biosci. Biotechnol. Biochem., 72(3), 833-841, 2008. The N-terminal partial amino acid sequence of this carbohydrate:acceptor oxidoreductase is given in sequence I.D. No. 1.

The carbohydrate:acceptor oxidoreductase comprises an amino acid sequence that has at least 60wt%, preferably at least 65wt%, or at least 70wt%, or at least 75wt%, or at least 80wt%, or at least 85wt%, or at least 90wt%, or at least 95wt% identity to sequence I.D. 1. Preferably the carbohydrate:acceptor oxidoreductase comprises the partial amino acid sequence of sequence I.D. 1.

The amino acid sequence identity is the relatedness between two amino acid sequences described by the parameter "identity". For purposes of the present invention, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277; http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

EXAMPLES

Example 1

A liquid laundry detergent composition comprises: 0.05wt% carbohydrate:acceptor oxidoreductase[*], 15wt% detersive surfactant, 3wt% fatty acid, 0.3wt% chelant, 0.2wt% brightener, 5wt% solvent, 0.5wt% perfume, buffer to pH 8.2, water and miscellaneous to 100wt%.

Example 2

A solid laundry detergent composition comprises: 0.05wt% carbohydrate:acceptor oxidoreductase[*], 10wt% detersive surfactant, 10wt% sodium carbonate, 0.3wt% chelant, 0.2wt% brightener, 0.5wt% perfume, moisture, sodium sulphate and miscellaneous to 100wt%.

[*] The carbohydrate:acceptor oxidoreductase is the wildtype from the organism *Paraconiothyrium* sp. disclosed in T.Kiryu et al, Biosci. Biotechnol. Biochem., 72(3), 833-841, 2008.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A detergent composition comprising carbohydrate:acceptor oxidoreductase comprising an amino acid sequence that has at least 60% identity to sequence I.D. 1.

2. A detergent composition according to claim 1, wherein the carbohydrate: acceptor oxidoreductase comprises an amino acid sequence that has at least 80% identity to sequence I.D. 1.

3. A detergent composition according to claim 1, wherein the carbohydrate:acceptor oxidoreductase is a parent or variant of the carbohydrate:acceptor oxidoreductase derived from the organism *Paraconiothyrium* sp.

4. A composition according to any preceding claim, wherein the composition is a laundry detergent composition.

5. A composition according to any preceding claim, wherein the composition is in liquid form.

6. A composition according to any preceding claim, wherein the composition is in solid form.

7. A composition according to any preceding claim, wherein the composition comprises detersive surfactant.

Europäisches
Patentamt

European
Patent Office

Office européen
des brevets

# EUROPEAN SEARCH REPORT

Application Number

EP 09 15 5707

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | KIRYU TAKAAKI ET AL: "Purification and characterization of a carbohydrate: Acceptor oxidoreductase from Paraconiothyrium sp that produces lactobionic acid efficiently" BIOSCIENCE BIOTECHNOLOGY AND BIOCHEMISTRY, vol. 72, no. 3, March 2008 (2008-03), pages 833-841, XP002539103 ISSN: 0916-8451 * the whole document * | 1-7 | INV. C12N9/04 C11D3/386 |
| A | XU F ET AL: "A novel carbohydrate:acceptor oxidoreductase from Microdochium nivale" EUROPEAN JOURNAL OF BIOCHEMISTRY, BLACKWELL PUBLISHING, BERLIN, DE, vol. 268, 1 January 2001 (2001-01-01), pages 1136-1142, XP002500983 ISSN: 0014-2956 * the whole document * | 1-7 | |
| A | WO 99/31990 A1 (NOVONORDISK AS [DK]) 1 July 1999 (1999-07-01) * page 25, line 31 - page 26, line 4; claims 1-28 * | 1-7 | TECHNICAL FIELDS SEARCHED (IPC) C12N C11D |
| A | WO 2006/131503 A2 (NOVOZYMES AS [DK]; LAURIDSEN CARSTEN [DK]; NIELSEN JESPER DUUS [DK]) 14 December 2006 (2006-12-14) * the whole document * | 1-7 | |

The present search report has been drawn up for all claims

| Place of search | Date of completion of the search | Examiner |
|---|---|---|
| The Hague | 29 July 2009 | Devijver, Kristof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 159 278 A1**

## ANNEX TO THE EUROPEAN SEARCH REPORT
## ON EUROPEAN PATENT APPLICATION NO.

EP 09 15 5707

29-07-2009

| Patent document cited in search report | | Publication date | Patent family member(s) | | Publication date |
|---|---|---|---|---|---|
| WO 9931990 | A1 | 01-07-1999 | AT | 293359 T | 15-05-2005 |
| | | | AU | 753578 B2 | 24-10-2002 |
| | | | AU | 1751899 A | 12-07-1999 |
| | | | CA | 2314996 A1 | 01-07-1999 |
| | | | CN | 1379989 A | 20-11-2002 |
| | | | CN | 1283082 A | 07-02-2001 |
| | | | DE | 69829878 D1 | 25-05-2005 |
| | | | DE | 69829878 T2 | 02-03-2006 |
| | | | EP | 1041890 A1 | 11-10-2000 |
| | | | ES | 2241189 T3 | 16-10-2005 |
| | | | JP | 2001526058 T | 18-12-2001 |
| WO 2006131503 | A2 | 14-12-2006 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T.Kiryu et al.** *Biosci. Biotechnol. Biochem.,* 2008, vol. 72 (3), 833-841
- **Needleman ; Wunsch.** *J. Mol. Biol.,* 1970, vol. 48, 443-453
- **Rice et al.** *Trends in Genetics,* 2000, vol. 16, 276-277, http://emboss.org

## SEQUENCE LISTING

<110> Procter & Gamble

<120> A detergent composition comprising carbohydrate:acceptor oxidoreductase

<130> CM3375F

<160> 1

<170> PatentIn version 3.5

<210> 1
<211> 40
<212> PRT
<213> Paraconiothyrium sp.

<220>
<221> misc_feature
<222> (6)..(6)
<223> Xaa can be any naturally occurring amino acid

<220>
<221> misc_feature
<222> (37)..(37)
<223> Xaa can be any naturally occurring amino acid

<400> 1

Ala Val Ile Asp Lys Xaa Leu Thr Asp Asp Gly Val Pro Val Asp Val
1               5                   10                  15

Lys Gly Ser Ala Trp Gly Asn Glu Asp Val Ala Pro Phe Asn Ile Arg
            20                  25                  30

Leu Pro Tyr Leu Xaa Thr Ala Ile
            35                  40